(19)

Europäisches
Patentamt
European
Patent Office
Office européen
des brevets

(11) **EP 3 376 404 A1**

(12) **EUROPEAN PATENT APPLICATION**
published in accordance with Art. 153(4) EPC

(43) Date of publication:
**19.09.2018 Bulletin 2018/38**

(51) Int Cl.:
**G06F 17/30** (2006.01)

(21) Application number: **16886527.7**

(22) Date of filing: **22.12.2016**

(86) International application number:
**PCT/JP2016/088375**

(87) International publication number:
**WO 2017/126288 (27.07.2017 Gazette 2017/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **18.01.2016 JP 2016007307**

(71) Applicants:
• **National Institute of Information
and Communication Technology
Tokyo 184-8795 (JP)**
• **NTT Data Corporation
Tokyo 135-6033 (JP)**
• **NTT Data Institute Of Management Consulting,
Inc.
Tokyo 102-0093 (JP)**

(72) Inventors:
• **NISHIMOTO Shinji
Koganei-shi
Tokyo 184-8795 (JP)**

• **NISHIDA Satoshi
Koganei-shi
Tokyo 184-8795 (JP)**
• **KASHIOKA Hideki
Koganei-shi
Tokyo 184-8795 (JP)**
• **YANO Ryo
Tokyo 135-6033 (JP)**
• **MAEDA Naoya
Tokyo 135-6033 (JP)**
• **KADO Masataka
Tokyo 135-6033 (JP)**
• **HAGIWARA Ippei
Tokyo 102-0093 (JP)**
• **IBARAKI Takuya
Tokyo 102-0093 (JP)**

(74) Representative: **Brevalex
95, rue d'Amsterdam
75378 Paris Cedex 8 (FR)**

(54) **VIEWING MATERIAL EVALUATION METHOD, VIEWING MATERIAL EVALUATION SYSTEM,
AND PROGRAM**

(57) A viewing material evaluating method includes: a brain activity measuring step of measuring a brain activity of a test subject who views a viewing material by using a brain activity measuring unit; a first matrix generating step of generating a first matrix estimating a semantic content of perception of the test subject on the basis of a measurement result acquired in the brain activity measuring step by using a first matrix generating unit; a second matrix generating step of generating a second matrix by performing natural language processing for text information representing a planning intention of the viewing material by using a second matrix generating unit; and a similarity calculating step of calculating similarity between the first matrix and the second matrix by using a similarity calculating unit.

FIG. 6

## Description

TECHNICAL FIELD

The present invention relates to a viewing material evaluating method, a viewing material evaluating system, and a program.

Priority is claimed on Japanese Patent Application No. 2016-7307, filed January 18, 2016, the content of which is incorporated herein by reference.

BACKGROUND ART

Conventionally, in a case in which a viewing material such as a commercial (hereinafter referred to as a CM) is evaluated, for example, as in an evaluation using a questionnaire, a subjective and qualitative evaluation is performed. A technology for estimating the semantic content of perception acquired by a test subject by measuring brain activity of the test subject under natural perception such as moving image viewing and analyzing measured information is known (for example, Patent Document 1). In the technology described in this Patent Document 1, words having high likelihoods are estimated from parts of speech including nouns, verbs, and adjectives, and thus an objective index can be acquired.

[Documents of the Prior Art]

[Patent Document]

[Patent Document 1] Japanese Unexamined Patent Application, First Publication No. 2015-077694

SUMMARY OF INVENTION

[Problems to be Solved by the Invention]

However, in a case in which a CM is evaluated using the technology of the description of Patent Document 1, for example, in a case in which an estimation result of "high class" is output, it is difficult to determine an evaluation corresponding to the intention of a CM producer. In this way, it is difficult to evaluate a viewing material objectively and qualitatively by using a conventional viewing material evaluating method.

The present invention is for solving the above-described problems, and an object thereof is to provide a viewing material evaluating method, a viewing material evaluating system, and a program capable of evaluating a viewing material objectively and qualitatively.

[Means for Solving the Problems]

In order to solve the problem described above, according to one aspect of the present invention, there is provided a viewing material evaluating method including: a brain activity measuring step of measuring brain activity of a test subject who views a viewing material by using a brain activity measuring unit; a first matrix generating step of generating a first matrix estimating semantic content of perception of the test subject on the basis of a measurement result acquired in the brain activity measuring step by using a first matrix generating unit; a second matrix generating step of generating a second matrix by performing natural language processing for text information representing a planning intention of the viewing material by using a second matrix generating unit; and a similarity calculating step of calculating similarity between the first matrix and the second matrix by using a similarity calculating unit.

In addition, according to one aspect of the present invention, there is provided a viewing material evaluating method in which, in the second matrix generating step of the viewing material evaluating method described above, the second matrix generating unit translates each of words acquired by dividing the text information into a matrix representing a position in a semantic space of a predetermined number of dimensions and generates the second matrix representing the center of the matrix.

Furthermore, according to one aspect of the present invention, there is provided a viewing material evaluating method in which, in the viewing material evaluating method described above, cut text information representing a planning intention of each cut included in a storyboard of the viewing material is included in the text information, in the first matrix generating step, the first matrix generating unit generates the first matrix for each cut, in the second matrix generating step, the second matrix generating unit generates the second matrix corresponding to the cut text information, and, in the similarity calculating step, the similarity calculating unit calculates the similarity for each cut.

In addition, according to one aspect of the present invention, there is provided a viewing material evaluating method in which, in the viewing material evaluating method described above, scene text information representing a planning intention of each scene included in the viewing material is included in the text information, in the first matrix generating step, the first matrix generating unit generates the first matrix for each scene, in the second matrix generating step, the second matrix generating unit generates the second matrix corresponding to the scene text information, and, in the similarity calculating step, the similarity calculating unit calculates the similarity for each scene.

Furthermore, according to one aspect of the present invention, there is provided a viewing material evaluating method in which, in the brain activity measuring step of the viewing material evaluating method described above, the brain activity measuring unit measures brain activity of the test subject for each predetermined time interval, in the first matrix generating step, the first matrix generating unit generates the first matrix for each predetermined time interval, and, in the similarity calculating step, the similarity calculating unit calculates similarity between a mean first matrix representing a mean of the first matrix in a period corresponding to the text information and the second matrix.

In addition, according to one aspect of the present invention, there is provided a viewing material evaluating method in which, in the viewing material evaluating method described above, overall intention text information representing an overall planning intention of the viewing material is included in the text information, in the brain activity measuring step, the brain activity measuring unit measures brain activity of the test subject for each predetermined time interval, in the first matrix generating step, the first matrix generating unit generates the first matrix for each predetermined time interval, in the second matrix generating step, the second matrix generating unit generates the second matrix corresponding to the overall intention text information, and, in the similarity calculating step, the similarity calculating unit calculates the similarity between the first matrix generated for each predetermined time interval and the second matrix corresponding to the overall intention text information.

Furthermore, according to one aspect of the present invention, there is provided a viewing material evaluating method in which, in the viewing material evaluating method described above, a training measuring step of measuring brain activity of the test subject viewing a training moving image at a predetermined time interval by using the brain activity measuring unit and a model generating step of generating an estimation model for estimating the first matrix from measurement results on the basis of a plurality of the measurement results acquired in the training measuring step and a plurality of third matrixes generated by performing natural language processing for description text describing each scene of the training moving image by using a model generating unit are further included, wherein, in the first matrix generating step, the first matrix generating unit generates the first matrix on the basis of the measurement result acquired in the brain activity measuring step and the estimation model.

In addition, according to one aspect of the present invention, there is provided a viewing material evaluating system including: a brain activity measuring unit measuring brain activity of a test subject who views a viewing material; a first matrix generating unit generating a first matrix estimating semantic content of perception of the test subject on the basis of a measurement result acquired by the brain activity measuring unit; a second matrix generating unit generating a second matrix by performing natural language processing for text information representing a planning intention of the viewing material; and a similarity calculating unit calculating similarity between the first matrix and the second matrix.

In addition, according to one aspect of the present invention, there is provided a program causing a computer to execute: a first matrix generating step of generating a first matrix estimating semantic content of perception of a test subject on the basis of a measurement result acquired by a brain activity measuring unit measuring brain activity of the test subject who views a viewing material; a second matrix generating step of generating a second matrix by performing natural language processing for text information representing a planning intention of the viewing material; and a similarity calculating step of calculating similarity between the first matrix and the second matrix.

[Advantageous Effects of the Invention]

According to the present invention, a viewing material can be evaluated objectively and qualitatively.

Brief Description of Drawings

Fig. 1 is a block diagram illustrating an example of an advertisement evaluating system according to a first embodiment.
Fig. 2 is a diagram illustrating an example of generation of an annotation vector according to the first embodiment.
Fig. 3 is a diagram illustrating the concept of a semantic space according to the first embodiment.
Fig. 4 is a diagram illustrating an example of an estimation model generating process according to the first embodiment.
Fig. 5 is a diagram illustrating an example of a CM moving image evaluating process according to the first embodiment.
Fig. 6 is a flowchart illustrating an example of the operation of the advertisement evaluating system according to the first embodiment.
Fig. 7 is a flowchart illustrating an example of an estimation model generating process according to the first embodiment.
Fig. 8 is a diagram illustrating an example of an evaluation result of the advertisement evaluating system according to the first embodiment.
Fig. 9 is a diagram illustrating an example of a CM moving image evaluating process according to a second embodiment.
Fig. 10 is a flowchart illustrating an example of the operation of the advertisement evaluating system according to the second embodiment.
Fig. 11 is a flowchart illustrating an example of the operation of an advertisement evaluating system according to a third embodiment.

EMBODIMENTS FOR CARRYING OUT THE INVENTION

Hereinafter, a viewing material evaluating system and a viewing material evaluating method according to one embodiment of the present invention will be described with reference to the drawings.

[First embodiment]

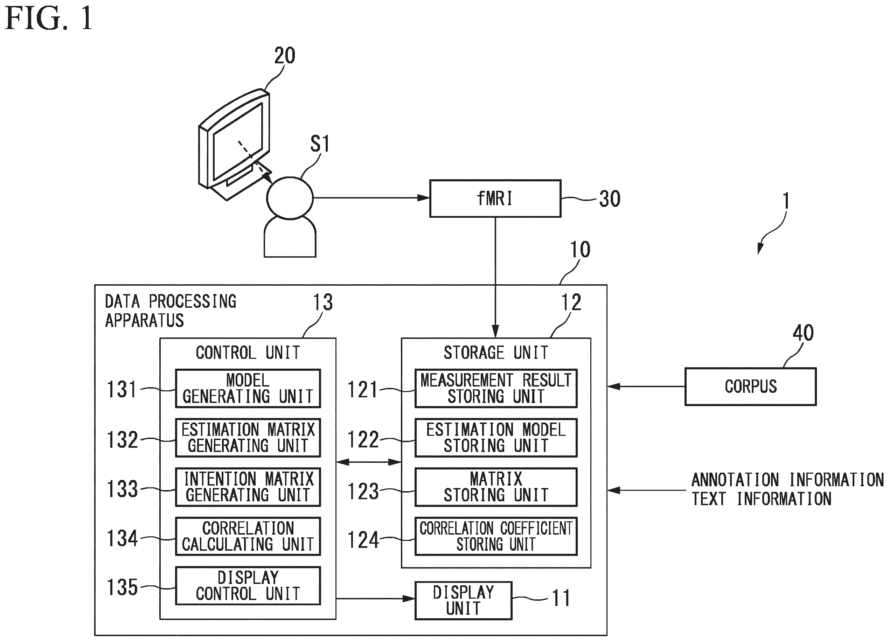

Fig. 1 is a block diagram illustrating an example of an advertisement evaluating system 1 according to a first embodiment.

As illustrated in Fig. 1, the advertisement evaluating system 1 includes a data processing apparatus 10, an image reproducing terminal 20, and a functional magnetic resonance imaging (fMRI) 30.

The advertisement evaluating system 1 according to this embodiment allows a test subject S1 to view a commercial moving image (CM moving image; commercial film (CF)) and evaluates the degree of reflection of the intention of a CM planning paper (the intention of a producer) objectively and qualitatively. In this embodiment, a CM moving image (advertisement moving image) is an example of a viewing material, and the advertisement evaluating system 1 will be described as an example of a viewing material evaluating system.

The image reproducing terminal 20, for example, is a terminal device including a liquid crystal display or the like and, for example, displays a moving image for training (training moving image), a CM moving image to be evaluated, or the like and allows a test subject S1 to view the displayed moving image. Here, the training moving image is a moving image including a wide variety of images.

The fMRI 30 (an example of a brain activity measuring unit) measures brain activity of the test subject S1 who has viewed an image (for example, a CM moving image or the like) displayed by the image reproducing terminal 20. The fMRI 30 outputs an fMRI signal (brain activity signal) that visualizes a hemodynamic reaction relating to brain activity of the test subject S1. The fMRI 30 measures the brain activity of the test subject S1 at the predetermined time interval (for example, a two-second interval) and outputs a measurement result to the data processing apparatus 10 as an fMRI signal.

The data processing apparatus 10 is a computer apparatus that evaluates a CM moving image on the basis of the measurement result for the brain activity of the test subject S1 measured by the fMRI 30. In addition, the data processing apparatus 10 generates an estimation model to be described later that is used for evaluating a CM moving image. The data processing apparatus 10 includes a display unit 11, a storage unit 12, and a control unit 13.

The display unit 11 (an example of an output unit) is, for example, a display device such as a liquid crystal display and displays information relating to various processes performed by the data processing apparatus 10. The display unit 11, for example, displays an evaluation result for the CM moving image.

The storage unit 12 stores various kinds of information used for various processes performed by the data processing apparatus 10. The storage unit 12 includes a measurement result storing unit 121, an estimation model storing unit 122, a matrix storing unit 123, and a correlation coefficient storing unit 124.

The measurement result storing unit 121 stores a measurement result acquired by the fMRI 30. The measurement result storing unit 121, for example, stores time information (or a sampling number) and a measurement result acquired by the fMRI 30 in association with each other.

The estimation model storing unit 122 stores an estimation model generated by a model generating unit 131 to be described later. Here, the estimation model is a model for estimating an estimation matrix A (first matrix) estimating semantic content of perception of the test subject S1 from a measurement result acquired by the fMRI 30. Details of the estimation matrix A will be described later.

The matrix storing unit 123 stores various kinds of matrix information used for evaluating a CM moving image. The matrix storing unit 123, for example, stores an object concept vector B (matrix B (second matrix)) generated from text information representing the intention of the plan of a CM, an estimation matrix A, and the like. Here, the object concept vector is a vector representing the concept of an object, in other words, the intention of the plan.

The correlation coefficient storing unit 124 (an example of a similarity storing unit) stores a correlation coefficient (r) corresponding to an evaluation result for a CM moving image. In other words, the correlation coefficient storing unit 124 stores a correlation coefficient (r) that is calculated by a correlation calculating unit 134 to be described later on the basis of the estimation matrix A and the object concept vector B (matrix B). The correlation coefficient storing unit 124, for example, stores time information (or a sampling number) and the correlation coefficient (r) in association with each other.

In addition, the similarity, for example, is calculated by using a Pearson correlation or a Euclidean distance.

The control unit 13, for example, is a processor including a central processing unit (CPU) or the like and integrally controls the data processing apparatus 10. The control unit 13 performs various processes performed by the data processing apparatus 10. For example, the control unit 13 generates an estimation model on the basis of a measurement result acquired by the fMRI 30 by allowing the test subject S1 to view a training moving image (training motion video) and an annotation vector that is vector data generated on the basis of data to which an annotation is assigned in advance for the training moving image. In addition, the control unit 13 generates a correlation coefficient (r) between a coordinate translation (matrix B) inside a semantic space used for evaluating a CM moving image and the matrix A on the basis of the measurement result acquired by the fMRI 30 by allowing the test subject S1 to view the CM moving image that is an evaluation target and text information representing the intention of the plan of the CM planning paper.

In addition, the control unit 13 includes a model generating unit 131, an estimation matrix generating unit 132, an intention matrix generating unit 133, a correlation calculating unit 134, and a display control unit 135.

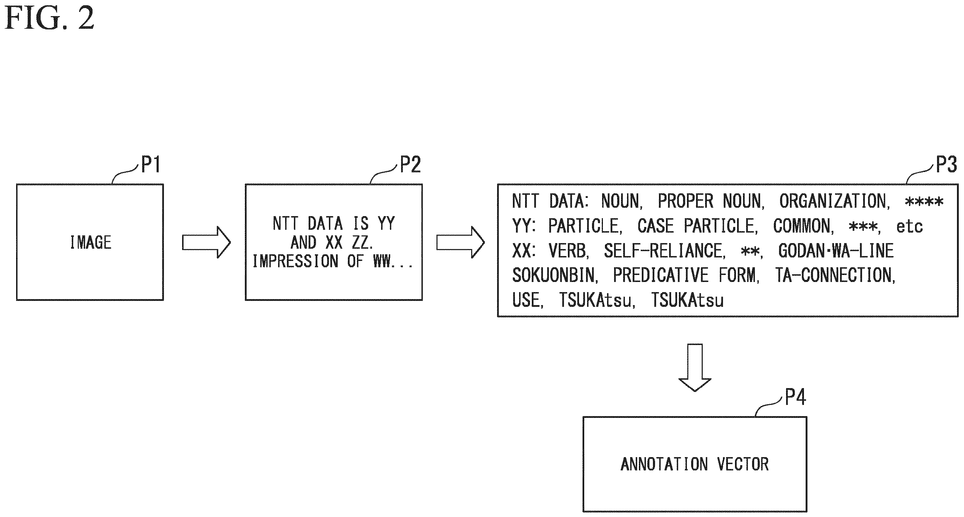

The model generating unit 131 generates an estimation model on the basis of a plurality of measurement results acquired by the fMRI 30 through measurements at the predetermined time interval by allowing the test subject S1 to view a training moving image and a plurality of annotation vectors (third matrixes) generated by performing natural language processing for description text describing each scene of the training moving image. The model generating unit 131, as illustrated in Fig. 2, generates an annotation vector (matrix) based on a still image of each scene of a training moving image or a moving image.

Fig. 2 is a diagram illustrating an example of generation of an annotation vector according to this embodiment.

Referring to Fig. 2, from an image PI, for example, a language description (annotation) P2 representing the impression of the image is generated. Text of the language description (annotation), for example, is text of a description of a scene overview, a feeling, or the like, and in order to avoid the bias of individual expressions describing an annotation, annotations described by a plurality of persons are used. The model generating unit 131, for example, performs a morpheme analysis P3 on the text of this language description (annotation), generates spaced word data to be decomposed into words and calculates an arithmetic mean of coordinate values of the words in an annotation vector space. Alternatively, coordinate values may be calculated for an aggregation of words, in other words, the whole text. Next, the model generating unit 131 performs natural language processing for the spaced word data by using a corpus 40 and generates an annotation vector space P4 such as Skipgram.

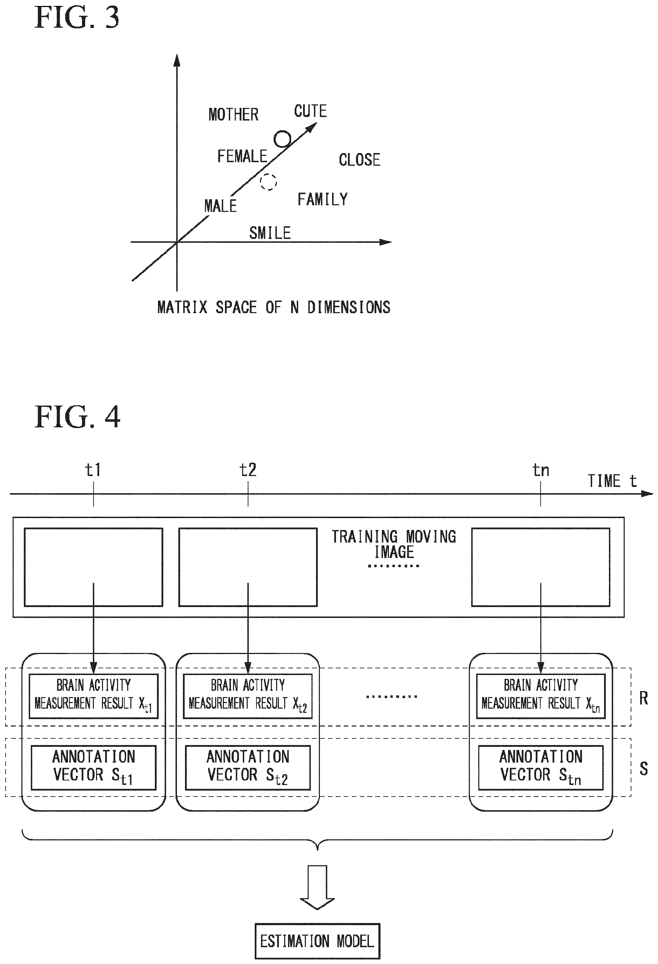

Here, the corpus 40, for example, is a database of a large amount of text data such as Wikipedia (registered trademark), newspaper articles, or the like. The model generating unit 131 performs natural language processing of such a large amount of text data for the spaced word data by using the corpus 40, thereby generating a word vector space. Here, the word vector space assigns coordinates in a same space, in other words, a vector to one word such as a noun, an adjective, a verb, or the like on the basis of appearance probabilities of words inside the corpus or the like. In this way, a word such as a noun representing the name of an object, an adjective representing an impression, or the like can be translated into coordinate values in a vector space (middle representation space) in which relations between words are represented as a matrix, and a relation between specific words can be specified as a distance between coordinates. Here, the vector space (middle representation space), for example, is a matrix space of a predetermined number of dimensions (N dimension) as illustrated in Fig. 3, and each word is assigned to corresponding coordinates of the matrix space (represented).

The model generating unit 131 translates each word included in the language description (annotation) representing the impression of an image into an annotation vector representing a position in the semantic space. The translation process is performed for each annotation described by a plurality of persons as a target. Thereafter, a vector representing the center (mean) of a plurality of annotation vectors acquired by performing the translation process is generated as an annotation vector representing the impression of the image. In other words, the model generating unit 131, for example, generates an annotation vector (third matrix) of the training moving image for every scene at two-second intervals and stores the generated annotation vectors in the matrix storing unit 123. The model generating unit 131, for example, stores time information (or a sampling number) and an annotation vector (third matrix) of each scene of the training moving image in the matrix storing unit 123 in association with each other.

In addition, the model generating unit 131, for example, acquires a measurement result of brain activity every two seconds that is acquired by the fMRI 30 when the training moving image displayed by the image reproducing terminal 20 is viewed by the test subject S1 and stores the measurement results in the measurement result storing unit 121. The model generating unit 131, for example, stores time information (or a sampling number) and a measurement result for brain activity acquired by the fMRI 30 on the basis of the training moving image in the measurement result storing unit 121 in association with each other.

In addition, the model generating unit 131 generates an estimation model on the basis of the measurement results acquired by the fMRI 30 on the basis of the training moving image and the annotation vector (third matrix) of each scene of the training moving image. Here, the estimation model is used for estimating an estimation matrix A that is semantic content of perception of the test subject S1 based on the measurement results of the brain activity.

Fig. 4 is a diagram illustrating an example of an estimation model generating process according to this embodiment.

As illustrated in Fig. 4, the model generating unit 131 acquires the measurement results ($X_{t1}$, $X_{t2}$, ⋯, $X_{tn}$) acquired by the fMRI 30 for the training moving image from the measurement result storing unit 121. In addition, the model generating unit 131 acquires the annotation vector ($S_{t1}$, $S_{t2}$, ⋯, $S_{tn}$) of each scene of the training moving image from the matrix storing unit 123. Here, when the measurement result ($X_{t1}$, $X_{t2}$, ⋯, $X_{tn}$) is denoted by a matrix R, and the annotation vector ($S_{t1}$, $S_{t2}$, ⋯, $S_{tn}$) is denoted by a matrix S, a general statistical model is represented by the following Equation (1).

$$S = f(R, \theta) \cdots (1)$$

Here, f() represents a function, and the variable θ represents a parameter.

In addition, for example, when Equation (1) described above is represented as a linear model, it is represented as in the following Equation (2).

$$S = R \times W \cdots (2)$$

Here, a matrix W represents a coefficient parameter in a linear model.

The model generating unit 131 generates an estimation model on the basis of Equation (2) described above by using the measurement result (matrix R) described above as a description variable and using the annotation vector (matrix S) as an objective variable. Here, a statistical model used for generating the estimation model may be a linear model (for example, a linear regression model or the like) or a non-linear model (for example, a non-linear regression model or the like).

For example, in a case in which the fMRI 30 measures brain activity of 60000 points at the interval of two seconds for a training moving image of two hours, the matrix R is a matrix of 3600 rows × 60000 digits. In addition, when the semantic space, for example, is a space of 1000 dimensions, the matrix S is a matrix of 3600 rows × 1000 digits, and the matrix W is a matrix of 60000 rows × 1000 digits. The model generating unit 131 generates an estimation model corresponding to the matrix W on the basis of the matrix R, the matrix S, and Equation (2). By using this estimation model, from a measurement result of 60000 points acquired by the fMRI 30, an annotation vector of 1000 dimensions can be estimated. The model generating unit 131 stores the generated estimation model in the estimation model storing unit 122.

In addition, the estimation model is preferably generated for each test subject S1, and the model generating unit 131 may store the generated estimation model and identification information used for identifying the test subject S1 in the estimation model storing unit 122 in association with each other.

The estimation matrix generating unit 132 (an example of a first matrix generating unit) generates an estimation matrix A (first matrix) estimating the semantic content of the perception of the test subject S1 on the basis of the measurement result acquired by the fMRI 30. The estimation matrix generating unit 132, for example, generates an estimation matrix A in which a measurement result is assigned to the semantic space illustrated in Fig. 3 on the basis of the measurement result acquired by the fMRI 30 by using the estimation model stored by the estimation model storing unit 122. The estimation matrix generating unit 132 stores the generated estimation matrix A in the matrix storing unit 123.

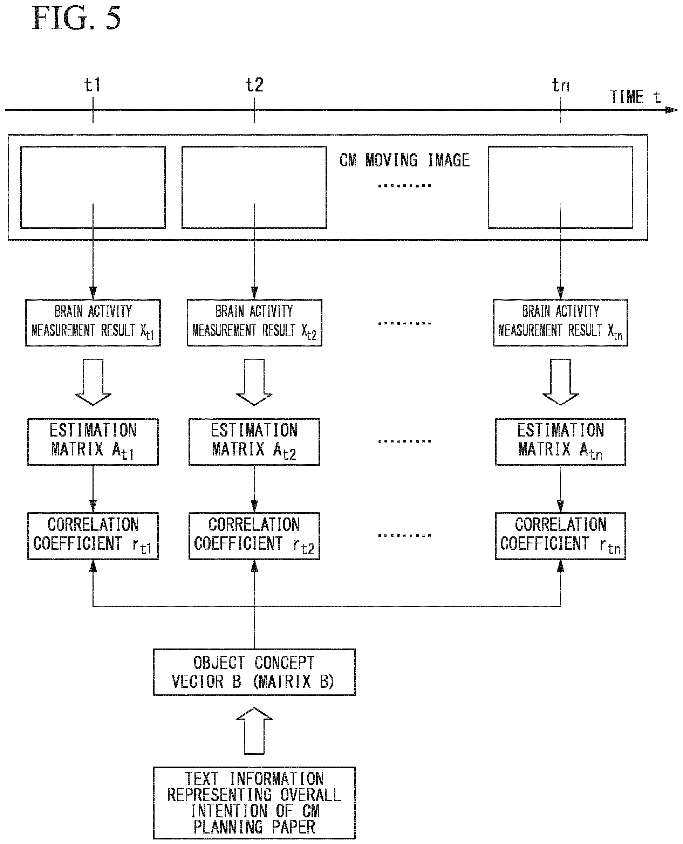

In addition, as illustrated in Fig. 5 to be described later, in a case in which the fMRI 30 outputs measurement results ($X_{t1}$, $X_{t2}$, $\cdots$, $X_{tn}$) at the predetermined time interval (time t1, time t2, $\cdots$, time tn), the estimation matrix generating unit 132 generates an estimation matrix A ($A_{t1}$, $A_{t2}$, $\cdots$, $A_{tn}$). In such a case, the estimation matrix generating unit 132 stores time information (time t1, time t2, $\cdots$, time tn) and the estimation matrix A ($A_{t1}$, $A_{t2}$, $\cdots$, $A_{tn}$) in the matrix storing unit 123 in association with each other.

The intention matrix generating unit 133 (an example of a second matrix generating unit) performs natural language processing for text information representing the intention of the plan of the CM moving image and generates an object concept vector B (matrix B (second matrix)) of the whole plan. For example, similar to the technique illustrated in Fig. 2, from the text information representing the overall intention of the plan such as a planning paper or the like of the CM moving image, an object concept vector B (matrix B) is generated. In other words, the intention matrix generating unit 133 translates the text information into spaced word data by performing a morpheme analysis thereof and performs natural language processing for words included in the spaced word data by using the corpus 40, thereby generating an object concept vector in units of words.

Then, the intention matrix generating unit 133 generates an object concept vector B (matrix B) of the whole plan of which the center is calculated on the basis of the generated object concept vector in units of words. In other words, the intention matrix generating unit 133 translates each word acquired by dividing the text information into a matrix (object concept vector) representing a position in the semantic space of a predetermined number of dimensions (for example, 1000 dimensions) and generates a matrix B representing the center of the matrix. The intention matrix generating unit 133 stores the generated object concept vector B (matrix B) in the matrix storing unit 123.

The correlation calculating unit 134 (an example of a similarity calculating unit) calculates a correlation (an example of similarity) between the estimation matrix A described above and the object concept vector B (matrix B). In other words, the correlation calculating unit 134, as illustrated in Fig. 5, calculates correlation coefficients $r(r_{t1}, r_{t2}, \cdots, r_{tn})$ between the estimation matrix A ($A_{t1}$, $A_{t2}$, $\cdots$, $A_{tn}$) generated at each predetermined time interval and the object concept vector B (matrix B) corresponding to text information representing the overall intention of the plan of the CM. The correlation calculating unit 134 stores the generated correlation coefficients r ($r_{t1}$, $r_{t2}$, $\cdots$, $r_{tn}$) and the time information (time t1, time t2, $\cdots$, time tn) in the correlation coefficient storing unit 124 in association with each other.

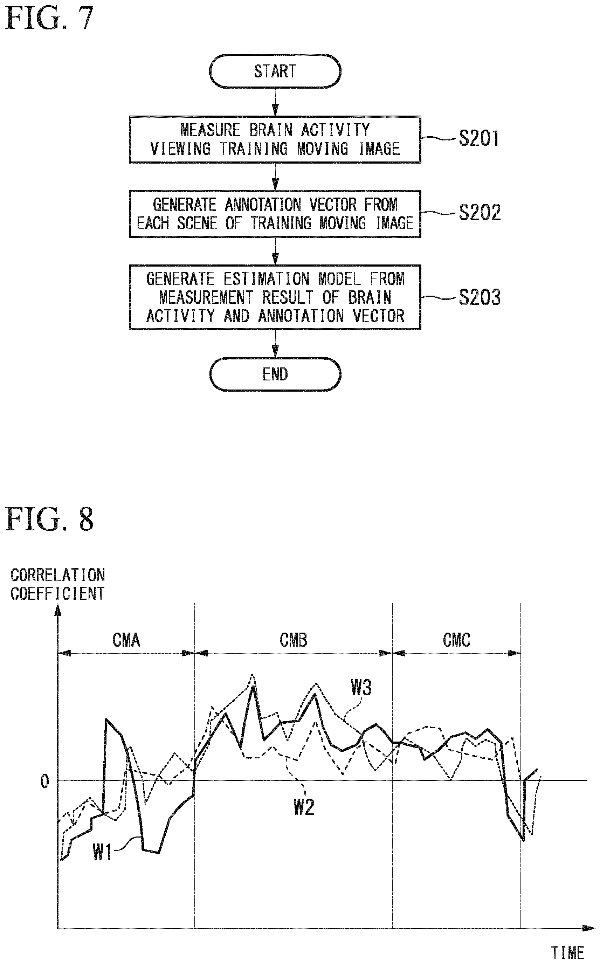

The display control unit 135 acquires the correlation coefficient r stored by the correlation coefficient storing unit 124, for example, generates a graph as illustrated in Fig. 8 to be described later, and displays a correlation between the overall intention of the plan of the CM and content perceived by a viewer that is output as a result of the brain activity of the viewer. The display control unit 135 displays (outputs) the generated graph of the correlation coefficient r on the display unit 11 as a result of the evaluation of the CM moving image.

Next, the operation of the advertisement evaluating system 1 according to this embodiment will be described with reference to the drawings.

Fig. 5 is a diagram illustrating an example of a CM moving image evaluating process according to this embodiment.

As illustrated in Fig. 5, in this embodiment, the overall intention text information representing the overall intention of the plan of the advertisement moving image is included in text information representing the intention of the plan of the CM. When the CM moving image displayed by the image reproducing terminal 20 is viewed by the test subject S1, the fMRI 30 measures the brain activity of the test subject S1 at each predetermined time interval (time t1, time t2, ···, time tn) and outputs measurement results ($X_{t1}$, $X_{t2}$, ···, $X_{tn}$).

In addition, the estimation matrix generating unit 132 generates an estimation matrix A ($A_{t1}$, $A_{t2}$, ···, $A_{tn}$) at each predetermined time interval from the measurement results ($X_{t1}$, $X_{t2}$, ···, $X_{tn}$) by using the estimation model stored by the estimation model storing unit 122. The intention matrix generating unit 133 generates an object concept vector B corresponding to the overall intention text information. Then, the correlation calculating unit 134 calculates correlation coefficients r ($r_{t1}$, $r_{t2}$, ···, $r_{tn}$) between the estimation matrix A ($A_{t1}$, $A_{t2}$, ···, $A_{tn}$) generated at each predetermined time interval and the object concept vector B (matrix B) corresponding to the overall intention text information.

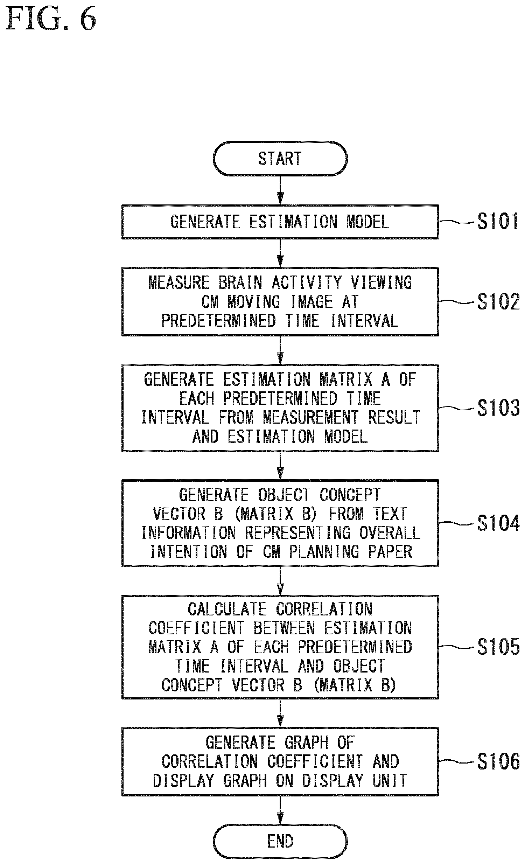

Fig. 6 is a flowchart illustrating an example of the operation of the advertisement evaluating system 1 according to this embodiment.

As illustrated in Fig. 6, the model generating unit 131 of the data processing apparatus 10 generates an estimation model (Step S101). In addition, a detailed process of generating an estimation model will be described later with reference to Fig. 7. The model generating unit 131 stores the generated estimation model in the estimation model storing unit 122.

Next, the fMRI 30 measures the brain activity of the test subject who has viewed the CM moving image at the predetermined time interval (Step S102). In other words, the fMRI 30 measures the brain activity of the test subject S1 who has viewed the CM moving image displayed by the image reproducing terminal 20, for example, at the interval of two seconds. The fMRI 30 outputs the measurement result ($X_{t1}$, $X_{t2}$, ···, $X_{tn}$) acquired through measurement to the data processing apparatus 10, and the data processing apparatus 10, for example, stores the measurement result in the measurement result storing unit 121.

Next, the estimation matrix generating unit 132 of the data processing apparatus 10 generates an estimation matrix A at each predetermined time interval from the measurement result and the estimation model (Step S103). The estimation matrix generating unit 132 generates an estimation matrix A (for example, $A_{t1}$, $A_{t2}$, ···, $A_{tn}$ illustrated in Fig. 5) for every two seconds from the measurement results for every two seconds stored by the measurement result storing unit 121 and the estimation model stored by the estimation model storing unit 122. The estimation matrix generating unit 132 stores the generated estimation matrix A in the matrix storing unit 123.

Next, the intention matrix generating unit 133 generates an object concept vector B (matrix B) from the text information (overall intention text information) representing the overall intention of the CM planning paper (Step S104). The intention matrix generating unit 133, for example, generates an object concept vector B (matrix B) by using a technique similar to the technique illustrated in Fig. 2. The intention matrix generating unit 133, for example, translates each word acquired by dividing the overall intention text information into a matrix (object concept vector) representing a position in a semantic space of a predetermined number of dimensions (for example, a semantic space of 1000 dimensions) and generates an object concept vector B (matrix B) representing the center of the matrix (object concept vector). The intention matrix generating unit 133 stores the generated object concept vector B (matrix B) in the matrix storing unit 123.

Next, the correlation calculating unit 134 of the data processing apparatus 10 calculates a correlation coefficient r between the estimation matrix A at each predetermined time interval and the object concept vector B (matrix B) (Step S105). The correlation calculating unit 134, for example, as illustrated in Fig. 5, calculates correlation coefficients r ($r_{t1}$, $r_{t2}$, ···, $r_{tn}$) between the estimation matrix A ($A_{t1}$, $A_{t2}$, ···, $A_{tn}$) for every two seconds stored by the matrix storing unit 123 and the object concept vector B (matrix B) stored by the matrix storing unit 123. The correlation calculating unit 134 stores the calculated correlation coefficients r ($r_{t1}$, $r_{t2}$, ···, $r_{tn}$) in the correlation coefficient storing unit 124.

Next, the data processing apparatus 10 generates a graph of the correlation coefficients r and displays the generated graph on the display unit 11 (Step SI06). In other words, the display control unit 135 of the data processing apparatus 10 acquires the correlation coefficients r ($r_{t1}$, $r_{t2}$, ···, $r_{tn}$) for every two seconds stored by the correlation coefficient storing unit 124 and, for example, generates a graph as illustrated in Fig. 8 to be described later. The display control unit 135 displays (outputs) the generated graph of the correlation coefficients r on the display unit 11 as a result of the evaluation of the CM moving image and ends the process.

In the flowchart of the advertisement evaluation (CM evaluation) described above, the process of Step S102 corresponds to the process of a brain activity measuring step, and the process of Step S103 corresponds to the process of a first matrix generating step. In addition, the process of Step S104 corresponds to the process of a second matrix generating step, and the process of Step S105 corresponds to the process of a correlation calculating step (a similarity calculating step).

Next, an estimation model generating process performed by the advertisement evaluating system 1 will be described with reference to Fig. 7.

Fig. 7 is a flowchart illustrating an example of an estimation model generating process according to this embodiment.

As illustrated in Fig. 7, the fMRI 30 measures brain activity of a test subject who has viewed the training moving image at the predetermined time interval (Step S201). In other words, the fMRI 30 measures the brain activity of the test subject S1 who has viewed the training moving image displayed by the image reproducing terminal 20, for example, at the interval of two seconds. The fMRI 30 outputs the measurement result $(X_{t1}, X_{t2}, \cdots, X_{tn})$ acquired through measurement to the data processing apparatus 10, and the model generating unit 131 of the data processing apparatus 10, for example, stores the measurement result in the measurement result storing unit 121.

Next, the model generating unit 131 generates an annotation vector that is vector data generated on the basis of data to which an annotation is assigned in advance for each scene of the training moving image (Step S202). The model generating unit 131, for example, generates an annotation vector $(S_{t1}, S_{t2}, \cdots, S_{tn})$ at the interval of two seconds (for each scene) by using the technique illustrated in Fig. 2. The model generating unit 131 stores the generated annotation vector $(S_{t1}, S_{t2}, \cdots, S_{tn})$ in the matrix storing unit 123.

Next, the model generating unit 131 generates an estimation model from the measurement result of the brain activity and the annotation vector (Step S203). In other words, the model generating unit 131 generates an estimation model, as illustrated in Fig. 4, by using Equation (2) using the measurement result $(X_{t1}, X_{t2}, \cdots, X_{tn})$ stored by the measurement result storing unit 121 as the matrix R and the annotation vector $(S_{t1}, S_{t2}, \cdots, S_{tn})$ stored by the matrix storing unit 123 as the matrix S. The model generating unit 131 stores the generated estimation model in the estimation model storing unit 122. After the process of Step S203, the model generating unit 131 ends the estimation model generating process.

In the flowchart of the estimation model generating process described above, the process of Step S201 corresponds to the process of a training measuring step, and the process of Steps S202 and S203 corresponds to the process of a generation step.

Next, an evaluation result of the advertisement evaluating system 1 according to this embodiment will be described with reference to Fig. 8.

Fig. 8 is a diagram illustrating an example of the evaluation result of the advertisement evaluating system 1 according to this embodiment.

Graphs illustrated in Fig. 8 represent graphs of evaluation results of an evaluation target CM (CMB), which is an evaluation target, and reference CM (CMA and CMC) for comparison. Here, the vertical axis represents the correlation coefficient r, and the horizontal axis represents the time.

In the example illustrated in Fig. 8, a comparison for three test subjects S1 is performed, a waveform W1 represents "test subject A", a waveform W2 represents "test subject B", and a waveform W3 represents "test subject C". A correlation coefficient here is an index representing the degree of reflection of the overall intention text information representing the overall intention of a CM planning paper (a CM panning paper of CMB) on a target CM moving image.

In the example illustrated in Fig. 8, a correlation coefficient for the evaluation target CMB tends to be higher than correlation coefficients for the reference CMs (CMA and CMC), which represents that the evaluation target CMB reflects the intention of the CM planning paper (the planning paper of the CMB) well.

As described above, the advertisement evaluating method (an example of a viewing material evaluating method) according to this embodiment includes a brain activity measuring step (Step S102 illustrated in Fig. 6), a first matrix generating step (Step S103 illustrated in Fig. 6), a second matrix generating step (Step S104 illustrated in Fig. 6), and a similarity calculating step (Step S105 illustrated in Fig. 6). In the brain activity measuring step, the fMRI 30 (brain activity measuring unit) measures the brain activity of a test subject S1 who has viewed a viewing material (CM moving image). In the first matrix generating step, the estimation matrix generating unit 132 (first matrix generating unit) generates an estimation matrix A (first matrix) used for estimating the semantic content of the perception of the test subject S1 on the basis of the measurement result acquired in the brain activity measuring step. In the second matrix generating step, the intention matrix generating unit 133 (second matrix generating unit) performs natural language processing for text information representing the intention of the plan of the advertisement moving image to generate an object concept vector B (the matrix B; the second matrix). In the similarity calculating step (correlation calculating step), the correlation calculating unit 134 calculates similarity (correlation coefficient r) between the estimation matrix A and the object concept vector B (matrix B).

In this way, the advertisement evaluating method according to this embodiment calculates a correlation coefficient r that is an index of an objective and qualitative CM evaluation of text information representing the intention of the plan of a viewing material (advertisement moving image), and accordingly, the viewing material (advertisement (CM)) can be evaluated objectively and qualitatively.

For example, in a case in which there are a CM (CMB) of a certain company and CMs (CMA and CMC) of competing companies, in an advertisement evaluating method according to this embodiment, the company can refer to other CMs (CMA and CMC) representing stronger reactions according to the intention of the plan of the CM of the own company than the CM (CMB) of the own company in a case in which CMs are present by comparing the evaluation results of the CM (CMA) of a competing company with the evaluation result of the CM (CMB) of the own company.

In addition, in the advertisement evaluating method according to this embodiment, it can be evaluated whether the intention of the plan at the time of ordering a CM to an advertisement agency is correctly delivered to viewers by comparing the object concept vector B (matrix B) on the basis of the overall intention text information according to the CM planning paper (for example, the planning paper of the CMB) with the estimation matrix A, for example, acquired by only viewing the CM (CMB) produced on the basis of the CM planning paper, and accordingly, the evaluation can be used as a material at the time of selecting an advertisement agent.

Furthermore, in this embodiment, in the second matrix generating step, the intention matrix generating unit 133 translates each word acquired by dividing text information into a matrix representing a position in the semantic space (see Fig. 3) of a predetermined number of dimensions (for example, 1000 dimensions) and generates an object concept vector B (matrix B) representing the center of the matrix.

Thus, according to the advertisement evaluating method of this embodiment, text information representing the intention of the plan of an advertisement moving image can be represented on a semantic space simply and appropriately, and accordingly, a relation between the intention of the plan according to the text information and the brain activity of the test subject S1 can be evaluated objectively and qualitatively.

In addition, in the text information representing the intention of the plan of the advertisement moving image, overall intention text information representing the overall intention of the plan of the advertisement moving image is included. In the brain activity measuring step, the fMRI 30 measures the brain activity of a test subject S1 at the predetermined time interval (for example, at the interval of two seconds). In the first matrix generating step, the estimation matrix generating unit 132 generates an estimation matrix A (for example, $A_{t1}$, $A_{t2}$, $\cdots$, $A_{tn}$) at each predetermined time interval. In the second matrix generating step, the intention matrix generating unit 133 generates an object concept vector B (matrix B) corresponding to the overall intention text information. In the similarity calculating step, the correlation calculating unit 134 calculates similarity (correlation coefficient r) between the estimation matrix A (for example, $A_{t1}$, $A_{t2}$, $\cdots$, $A_{tn}$) generated at each predetermined time interval and the object concept vector B (matrix B) corresponding to the overall intention text information.

In this way, in the advertisement evaluating method according to this embodiment, similarity (correlation coefficient r) corresponding to the overall intention text information of each predetermined time interval is calculated, and accordingly, the degree of reflection of the overall intention of the plan of the CM on the CM moving image can be evaluated at each predetermined time interval.

In addition, the advertisement evaluating method according to this embodiment includes the training measuring step and the generation step. In the training measuring step, the fMRI 30 measures the brain activity of the test subject S1 who has viewed the training moving image at the predetermined time interval (for example, at the interval of two seconds). In the model generating step, the model generating unit 131 generates an estimation model for estimating the estimation matrix A from the measurement result X on the basis of a plurality of measurement results (for example, $X_{t1}$, $X_{t2}$, $\cdots$, $X_{tn}$ illustrated in Fig. 4) acquired in the training measuring step and a plurality of annotation vectors S (the third matrix; for example, $S_{t1}$, $S_{t2}$, $\cdots$, $S_{tn}$) generated by performing natural language processing for a description text describing each scene of the training moving image. Then, in the first matrix generating step, the estimation matrix generating unit 132 generates an estimation matrix A on the basis of the measurement result X acquired in the brain activity measuring step and the estimation model.

In this way, according to the advertisement evaluating method of this embodiment, an estimation model can be generated, and, for example, an estimation model that is optimal for each test subject S1 can be generated. Thus, according to the advertisement evaluating method of this embodiment, the advertisement (CM) can be objectively and qualitatively evaluated with high accuracy for each test subject S1.

In addition, the advertisement evaluating system 1 (an example of a viewing material evaluating system) according to this embodiment includes the fMRI 30, the estimation matrix generating unit 132, the intention matrix generating unit 133, and the correlation calculating unit 134. The fMRI 30 measures the brain activity of a test subject S1 who has viewed a CM moving image. The estimation matrix generating unit 132 generates an estimation matrix A (first matrix) estimating the semantic content of the perception of the test subject S1 on the basis of the measurement result acquired by the fMRI 30. The intention matrix generating unit 133 performs natural language processing for text information representing the intention of the plan of the CM moving image and generates an object concept vector B (matrix B (second matrix)). Then, the correlation calculating unit 134 calculates similarity (correlation coefficient r) between the estimation matrix A and the object concept vector B (matrix B).

In this way, the advertisement evaluating system 1 according to this embodiment, similar to the advertisement evaluating method according to this embodiment, can evaluate an advertisement (CM) objectively and qualitatively.

In addition, the data processing apparatus 10 (an example of a viewing material evaluating apparatus) according to this embodiment includes the estimation matrix generating unit 132, the intention matrix generating unit 133, and the correlation calculating unit 134. The estimation matrix generating unit 132 generates an estimation matrix A (first matrix) estimating the semantic content of the perception of the test subject S1 on the basis of the measurement result acquired by the fMRI 30 measuring the brain activity of the test subject S1 who has viewed the CM moving image. The intention matrix generating unit 133 performs natural language processing for text information representing the intention of the plan of the CM moving image and generates an object concept vector B (matrix B (second matrix)). Then, the correlation calculating unit 134 calculates similarity (correlation coefficient r) between the estimation matrix A and the object concept vector B (matrix B).

In this way, the data processing apparatus 10 (viewing material evaluating apparatus) according to this embodiment, similar to the advertisement evaluating method and the advertisement evaluating system 1 according to this embodiment, can evaluate an advertisement (CM) objectively and qualitatively.

[Second Embodiment]

Next, an advertisement evaluating system 1 and an advertisement evaluating method according to a second embodiment will be described with reference to the drawings.

The configuration of the advertisement evaluating system 1 according to this embodiment is similar to that of the first embodiment illustrated in Fig 1, and the description thereof will not be presented here.

In this embodiment, text information (cut text information) representing the intention of the plan is extracted for each cut of the storyboard that is an example of a planning paper of a CM, and the CM image is evaluated for each cut of the storyboard, which is different from the first embodiment.

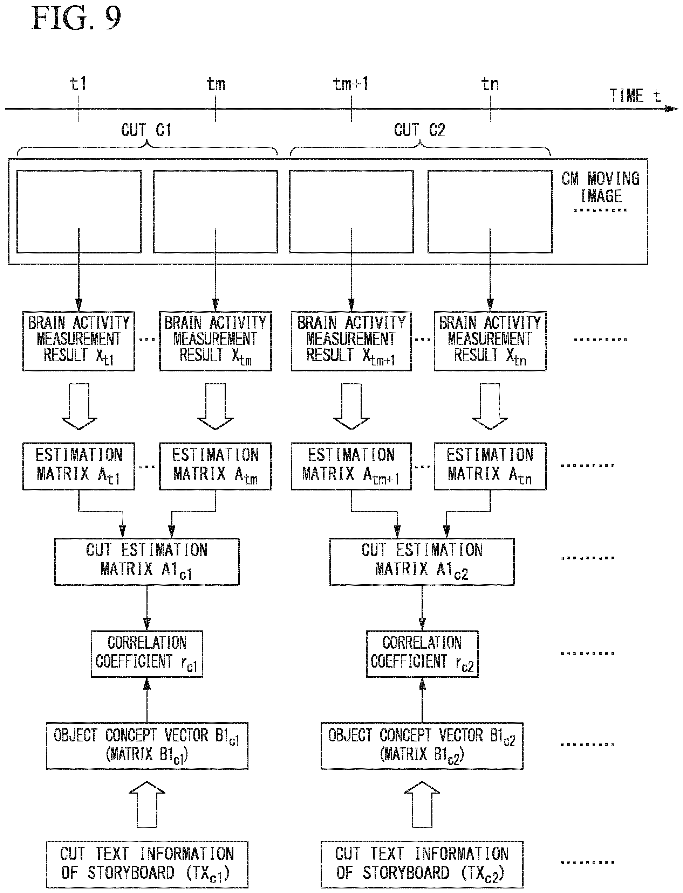

Fig. 9 is a diagram illustrating an example of a CM moving image evaluating process according to the second embodiment.

In Fig. 9, each cut of a storyboard corresponds to a plurality of number of times of measurement performed by a fMRI 30. For example, a cut C1 corresponds to measurement of time t1 to time tm using the fMRI 30, and a cut C2 corresponds to measurement of time tm+1 to time tn using the fMRI 30. In addition, a text representing the intention of the plan corresponding to the cut C1 of the storyboard is cut text information ($TX_{c1}$), and a text representing the intention of the plan corresponding to the cut C2 of the storyboard is cut text information ($TX_{c2}$).

In this embodiment, an estimation matrix generating unit 132 generates an estimation matrix A1($A1_{c1}$, $A1_{c2}$, ⋯) for each cut. For example, as illustrated in Fig.

9, the estimation matrix generating unit 132 generates an estimation matrix A ($A_{c1}$ to $A_{cm}$) corresponding to measurement results ($X_{c1}$ to $X_{cm}$) using the fMRI 30 by using an estimation model stored by an estimation model storing unit 122. In addition, the estimation matrix generating unit 132 generates a mean estimation matrix A1 (mean first matrix) representing the mean of the estimation matrix A in a period corresponding to the cut text information. For example, for the cut C1 corresponding to time t1 to time tm, the estimation matrix generating unit 132 generates a mean estimation matrix A1c1 representing the mean of the estimation matrixes ($A_{c1}$ to $A_{cm}$). In addition, for example, for the cut C2 corresponding to time tm+1 to time tn, the estimation matrix generating unit 132 generates a mean estimation matrix $A1_{c2}$ representing the mean of the estimation matrixes ($A_{cm+1}$ to $A_{cn}$).

Furthermore, the intention matrix generating unit 133 generates an object concept vector B (matrix B1) for each cut text information. The intention matrix generating unit 133, similar to the technique illustrated in Fig. 2 described above, generates an object concept vector (a matrix $B1_{c1}$, a matrix $B1_{c2}$, ⋯) for each cut text information.

Then, the correlation calculating unit 134 calculates a correlation coefficient r for each cut. In addition, in this embodiment, correlation coefficients r ($r_{c1}$, $r_{c2}$, ⋯) between the mean estimation matrix A1 representing the mean of the estimation matrix A in a period corresponding to the cut text information and a second matrix.

In this way, in this embodiment, in text information representing the intention of the plan of a CM planning paper, cut text information (for example, $TX_{c1}$, $TX_{c2}$, ⋯) representing the intention of the plan for each cut included in the storyboard of a CM moving image is included. The estimation matrix generating unit 132 generates an estimation matrix A1 for each cut, and the intention matrix generating unit 133 generates an object concept vector B1 (matrix B1) for each cut text information, and the correlation calculating unit 134 calculates a correlation coefficient r for each cut.

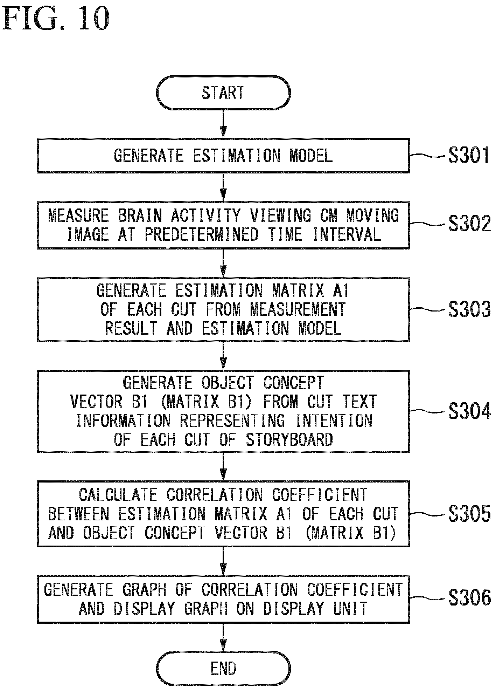

Next, the operation of the advertisement evaluating system 1 according to this embodiment will be described with reference to Fig. 10.

Fig. 10 is a flowchart illustrating an example of the operation of the advertisement evaluating system 1 according to this embodiment.

As illustrated in Fig. 10, a model generating unit 131 of a data processing apparatus 10 generates an estimation model (Step S301). Here, an estimation model generating process using the model generating unit 131 is similar to that according to the first embodiment. The model generating unit 131 stores the generated estimation model in the estimation model storing unit 122.

Next, the fMRI 30 measures the brain activity of a test subject who has viewed a CM moving image at the predetermined time interval (Step S302). In other words, the fMRI 30 measures the brain activity of the test subject S1 who has viewed the CM moving image displayed by the image reproducing terminal 20, for example, at the interval of two seconds. The fMRI 30 outputs the measurement result $(X_{t1}, X_{t2}, \cdots, X_{tn})$ acquired through measurement to the data processing apparatus 10, and the data processing apparatus 10, for example, stores the measurement result in the measurement result storing unit 121.

Next, the estimation matrix generating unit 132 of the data processing apparatus 10 generates an estimation matrix A1 for each cut from the measurement result and the estimation model (Step S303). The estimation matrix generating unit 132, as illustrated in Fig. 9, generates an estimation matrix A for every two seconds from the measurement results for every two seconds stored by the measurement result storing unit 121 and the estimation model stored by the estimation model storing unit 122 and generates a mean estimation matrix A1 representing the mean of the estimation matrix A in a period corresponding to the cut text information. The estimation matrix generating unit 132 stores the generated estimation matrix A1 in the matrix storing unit 123.

Next, the intention matrix generating unit 133 generates an object concept vector B1 (matrix B1) from cut text information representing the intention for each cut of the storyboard (Step S304). The intention matrix generating unit 133, for example, generates an object concept vector B1 (matrix B1) for each cut of the storyboard by using a technique similar to the technique illustrated in Fig. 2. The intention matrix generating unit 133 stores the generated object concept vector B1 (matrix B1) in the matrix storing unit 123.

Next, the correlation calculating unit 134 of the data processing apparatus 10 calculates a correlation coefficient r between the estimation matrix A1 for each cut and the object concept vector B1 (matrix B1) (Step S305). The correlation calculating unit 134, for example, as illustrated in Fig. 9, calculates correlation coefficients r $(r_{c1}, r_{c2}, \cdots)$ between the estimation matrix A1 for each cut stored by the matrix storing unit 123 and the object concept vector B1 (matrix B1) for each cut stored by the matrix storing unit 123. The correlation calculating unit 134 stores the calculated correlation coefficients r $(r_{c1}, r_{c2}, \cdots)$ in the correlation coefficient storing unit 124.

Next, the data processing apparatus 10 generates a graph of the correlation coefficients r and displays the generated graph on the display unit 11 (Step S306). In other words, the display control unit 135 of the data processing apparatus 10 acquires the correlation coefficients r $(r_{c1}, r_{c2}, \cdots)$ for each cut stored by the correlation coefficient storing unit 124 and, for example, generates a graph of the correlation coefficient r for the cut of the storyboard. The display control unit 135 displays (outputs) the generated graph of the correlation coefficients r on the display unit 11 as a result of the evaluation of the CM moving image and ends the process.

In the flowchart of the advertisement evaluation (CM evaluation) described above, the process of Step S302 corresponds to the process of a brain activity measuring step, and the process of Step S303 corresponds to the process of a first matrix generating step. In addition, the process of Step S304 corresponds to the process of a second matrix generating step, and the process of Step S305 corresponds to the process of a correlation calculating step (a similarity calculating step).

As described above, according to the advertisement evaluating method of this embodiment, cut text information representing the intention of the plan of each cut included in the storyboard of a CM moving image is included in the text information. In the first matrix generating step, the estimation matrix generating unit 132 generates an estimation matrix A1 for each cut of the storyboard, and, in the second matrix generating step, the intention matrix generating unit 133 generates an object concept vector B1 (matrix B1) corresponding to the cut text information. Then, in the correlation calculating step (similarity calculating step), the correlation calculating unit 134 calculates similarity (the correlation coefficient r) for each cut of the storyboard.

In this way, the advertisement evaluating method according to this embodiment can evaluate the advertisement (CM) for each cut of the storyboard objectively and qualitatively. For example, according to the advertisement evaluating method of this embodiment, for the intention of the production of the cut of the storyboard, the impression of the CM moving image can be evaluated objectively and qualitatively. Therefore, according to the advertisement evaluating method of this embodiment, an advertisement (CM) can be evaluated in more detail.

In addition, according to this embodiment, in the brain activity measuring step, the fMRI 30 measures the brain activity of a test subject S1 at a predetermined time interval (for example, at the interval of two seconds), and, in the first matrix generating step, the estimation matrix generating unit 132 generates an estimation matrix A at a predetermined time interval (for example, at the interval of two seconds). Then, the estimation matrix generating unit 132 generates a mean estimation matrix A1 representing the mean of the estimation matrix A in a period (a period corresponding to the cut) corresponding to text information (cut text information) for each cut as an estimation matrix. Then, in the correlation calculating step (similarity calculating step), the correlation calculating unit 134 calculates a correlation coefficient r between the mean estimation matrix A1 representing the mean of the estimation matrix A in the period corresponding to the text information and the object concept vector B1 (matrix B1) for each cut.

In this way, according to the advertisement evaluating method of this embodiment, an estimation matrix A1 (mean estimation matrix) for each cut can be generated using a simple technique, and a CM moving image can be appropriately evaluated for each cut of the storyboard.

[Third embodiment]

Next, an advertisement evaluating system 1 and an advertisement evaluating method according to a third embodiment will be described with reference to the drawings.

The configuration of the advertisement evaluating system 1 according to this embodiment is similar to that of the first embodiment illustrated in Fig 1, and the description thereof will not be presented here.

In this embodiment, text information (scene text information) representing the intention of the plan is extracted for each scene of the CM moving image, and the CM image is evaluated for each scene of the CM moving image, which is different from the first and second embodiments. Here, a scene of a CM moving image is a partial moving image configured by a plurality of cuts (at least one cut).

In the advertisement evaluating system 1 and the advertisement evaluating method according to this embodiment, the cut of the storyboard according to the second embodiment is replaced with a scene, which is different from the second embodiment.

In this embodiment, for example, an estimation matrix generating unit 132 generates an estimation matrix A2 for each scene, and an intention matrix generating unit 133 generates an object concept vector B2 for each scene text information. Then, a correlation calculating unit 134 calculates similarity (correlation coefficient r) for each scene.

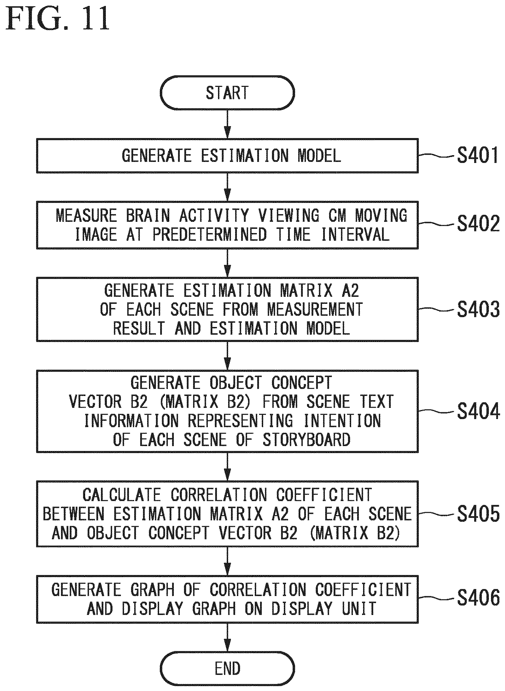

Next, the operation of the advertisement evaluating system 1 according to this embodiment will be described with reference to Fig. 11.

Fig. 11 is a flowchart illustrating an example of the operation of the advertisement evaluating system 1 according to this embodiment.

As illustrated in Fig. 11, a model generating unit 131 of a data processing apparatus 10 generates an estimation model (Step S401). Here, an estimation model generating process using the model generating unit 131 is similar to that according to the first embodiment. The model generating unit 131 stores the generated estimation model in the estimation model storing unit 122.

Next, the fMRI 30 measures the brain activity of a test subject who has viewed a CM moving image at the predetermined time interval (Step S402). In other words, the fMRI 30 measures the brain activity of the test subject S1 who has viewed the CM moving image displayed by the image reproducing terminal 20, for example, at the interval of two seconds. The fMRI 30 outputs the measurement result $(X_{t1}, X_{t2}, \cdots, X_{tn})$ acquired through measurement to the data processing apparatus 10, and the data processing apparatus 10, for example, stores the measurement result in the measurement result storing unit 121.

Next, the estimation matrix generating unit 132 of the data processing apparatus 10 generates an estimation matrix A2 for each scene from the measurement result and the estimation model (Step S403). The estimation matrix generating unit 132 generates an estimation matrix A for every two seconds from the measurement results for every two seconds stored by the measurement result storing unit 121 and the estimation model stored by the estimation model storing unit 122 and generates a mean estimation matrix A2 representing the mean of the estimation matrix A in a period corresponding to the scene text information. The estimation matrix generating unit 132 stores the generated estimation matrix A2 in the matrix storing unit 123.

Next, the intention matrix generating unit 133 generates an object concept vector B2 (matrix B2) from scene text information representing the intention of the plan for each scene (Step S404). The intention matrix generating unit 133, for example, generates an object concept vector B2 (matrix B2) for each scene by using a technique similar to the technique illustrated in Fig. 2. The intention matrix generating unit 133 stores the generated object concept vector B2 (matrix B2) in the matrix storing unit 123.

Next, the correlation calculating unit 134 of the data processing apparatus 10 calculates a correlation coefficient r between the estimation matrix A2 for each cut and the object concept vector B2 (matrix B2) (Step S405). The correlation calculating unit 134 calculates a correlation coefficient r between the estimation matrix A2 for each cut stored by the matrix storing unit 123 and the object concept vector B2 (matrix B2) for each cut stored by the matrix storing unit 123. The correlation calculating unit 134 stores the calculated correlation coefficient r in the correlation coefficient storing unit 124.

Next, the data processing apparatus 10 generates a graph of the correlation coefficients r and displays the generated graph on the display unit 11 (Step S406). In other words, the display control unit 135 of the data processing apparatus 10 acquires the correlation coefficient r for each scene stored by the correlation coefficient storing unit 124 and, for example, generates a graph of the correlation coefficient r for the scene of the CM moving image. The display control unit 135 displays (outputs) the generated graph of the correlation coefficients r on the display unit 11 as a result of the evaluation of the CM moving image and ends the process.

In the flowchart of the advertisement evaluation (CM evaluation) described above, the process of Step S402 corresponds to the process of a brain activity measuring step, and the process of Step S403 corresponds to the process of a first matrix generating step. In addition, the process of Step S404 corresponds to the process of a second matrix generating step, and the process of Step S405 corresponds to the process of a correlation calculating step (a similarity calculating step).

As described above, according to the advertisement evaluating method of this embodiment, scene text information representing the intention of the plan of each scene included in a CM moving image is included in the text information. In the first matrix generating step, the estimation matrix generating unit 132 generates an estimation matrix A2 for each scene, and, in the second matrix generating step, the intention matrix generating unit 133 generates an object concept vector B2 (matrix B2) corresponding to the cut text information. Then, in the correlation calculating step (similarity calculating step), the correlation calculating unit 134 calculates similarity (the correlation coefficient r) for each cut of the storyboard.

In this way, the advertisement evaluating method according to this embodiment can evaluate the advertisement (CM) for each scene objectively and qualitatively. For example, according to the advertisement evaluating method of this embodiment, for the intention of the production of the scene, the impression of the CM moving image can be evaluated objectively and qualitatively. Therefore, according to the advertisement evaluating method of this embodiment, an advertisement (CM) can be evaluated in further more detail than the second embodiment. For example, while the intention of the plan of the CM is evaluated to be reflected on the whole as the evaluation of the whole CM or the evaluation of each cut, by evaluating a result of the perception of a viewer for a specific scene (for example, the expression or the behavior of an appearing actor) in detail, the effect of the CM can be improved.

In addition, according to this embodiment, in the brain activity measuring step, the fMRI 30 measures the brain activity of a test subject S1 at the predetermined time interval (for example, at the interval of two seconds), and, in the first matrix generating step, the estimation matrix generating unit 132 generates an estimation matrix A at the predetermined time interval (for example, at the interval of two seconds). Then, the estimation matrix generating unit 132 generates a mean estimation matrix A2 representing the mean of the estimation matrix A in a period (a period corresponding to the scene) corresponding to text information (scene text information) for each scene as an estimation matrix. Then, in the correlation calculating step (similarity calculating step), the correlation calculating unit 134 calculates a correlation coefficient r between the mean estimation matrix A2 representing the mean of the estimation matrix A in the period corresponding to the text information and the object concept vector B2 (matrix B2) for each scene.

In this way, according to the advertisement evaluating method of this embodiment, an estimation matrix A2 (mean estimation matrix) for each scene can be generated using a simple technique, and an evaluation of each scene of the CM moving image can be appropriately performed.

The present invention is not limited to each of the embodiments described above, and a change can be made in a range not departing from the concept of the present invention.

For example, while an example in which each of the embodiments described above is independently performed has been described, the embodiments may be combined together.

In addition, in each of the embodiments described above, while an example in which the data processing apparatus 10 includes the model generating unit 131 generating an estimation model has been described, the configuration is not limited thereto. Thus, an estimation model generated in advance may be stored in the estimation model storing unit 122 without including the model generating unit 131. Furthermore, an apparatus such as an analysis apparatus that is separate from the data processing apparatus 10 may be configured to include the model generating unit 131.

In addition, in each of the embodiments described above, while an example in which the model generating unit 131 generates an estimation model by using the center of the annotation vector in units of words as the annotation vector of a scene has been described, the method of generating an estimation model is not limited thereto. Thus, an estimation model may be configured to be generated by using the annotation vector in units of words.

Furthermore, in the first embodiment described above, while an example in which a correlation coefficient r between the estimation matrix A of a predetermined time interval and the object concept vector B (matrix B) corresponding to the overall intention text information is calculated and used for the evaluation, a correlation coefficient r between a mean estimation matrix of the estimation matrix A of a predetermined time interval over all the period and an object concept vector B (matrix B) corresponding to the overall intention text information may be calculated and used for the evaluation.

In addition, in each of the embodiments described above, while an example in which a CM is evaluated by causing a test subject S1 to view the CM moving image as an example of the evaluation of a viewing material has been described, the evaluation may be performed by causing a test subject S1 to view an illustration or a still screen of a storyboard. For example, in a case in which there are a plurality of storyboard plans in a planning stage before the production of a CM or the like, the fMRI 30 may measure the brain activity of the test subject S1 who has viewed still screens of each storyboard plan, the estimation matrix generating unit 132 may generate an estimation matrix for a plurality of still screens, and the correlation calculating unit 134 may calculate a correlation coefficient on the basis of the estimation matrix. In such a case, a storyboard plan that is closest to the conditions (the intention of production) of a planning paper can be evaluated before the production of a CM. In addition, a storyboard plan that is closer to the conditions (the intention of production) of the planning paper can be selected from among a plurality of storyboards. In this way, a viewing material that is the viewing material to be viewed and evaluated by the test subject S1 and is an evaluation target, in addition to a moving image such as a CM moving image, includes a still screen, a printed material (for example, an advertisement, a leaflet, a web page or the like) using various media, and the like.

In addition, in each of the embodiments described above, while an example in which a correlation coefficient (r) representing a correlation is used as an example of the similarity has been described, the similarity is not limited to the correlation coefficient. For example, each of the embodiments described above may use another index representing the similarity, a semantic distance (statistical distance), or the like.

Furthermore, in each of the embodiments described above, while an example in which the center (mean) of the object concept vector in units of words or a mean of the object concept vectors of a predetermined time interval is used for the generation of an object concept vector for text information or the generation of an object concept vector for each scene or cut has been described, the technique is not limited thereto, and any other technique using a distribution (dispersion) of a vector or the like may be used.

In addition, in the second and third embodiments described above, while an example in which a mean over a period corresponding to a cut (or a scene) of the object concept vector of each predetermined time interval is used for the generation of an object concept vector for each cut (or scene), the technique is not limited thereto. For example, the estimation matrix generating unit 132 may calculate a mean value over a period corresponding to a cut (or scene) of the measurement result acquired by the fMRI 30 of each predetermined time interval and generate an object concept vector for each cut (or scene) from the mean value of the measurement results.

In addition, in each of the embodiments described above, while an example in which the data processing apparatus 10 includes the display unit 11 as an example of an output unit and outputs an evaluation result to the display unit 11 has been described, the output unit is not limited thereto. For example, the output unit may be a printer, an interface unit outputting the evaluation result as a file, or the like. Furthermore, a part or the whole of the storage unit 12 may be arranged outside the data processing apparatus 10.

In addition, each configuration included in the data processing apparatus 10 described above includes an internal computer system. Then, by recording a program used for realizing the function of each configuration included in the data processing apparatus 10 described above on a computer-readable recording medium and causing the computer system to read and execute the program recorded on this recording medium, the process of each configuration included in the data processing apparatus 10 described above may be performed. Here, "the computer system is caused to read and execute the program recorded on the recording medium" includes a case in which the computer system is causes to install the program in the computer system. The "computer system" described here includes an OS and hardware such as peripherals.

In addition, the "computer system" may include a plurality of computer apparatuses connected through a network including the Internet, a WAN, a LAN or a communication line such as a dedicated line. Furthermore, the "computer-readable recording medium" represents a portable medium such as a flexible disc, a magneto-optical disk, a ROM, or a CD-ROM or a storage device such as a hard disk built in the computer system. In this way, the recording medium in which the program is stored may be a non-transient recording medium such as a CD-ROM.

In addition, the recording medium includes a recording medium installed inside or outside that is accessible from a distribution server for distributing the program. Furthermore, a configuration in which the program is divided into a plurality of parts, and the parts are downloaded at different timings and then are combined in each configuration included in the data processing apparatus 10 may be employed, and distribution servers distributing the divided programs may be different from each other. In addition, the "computer-readable recording medium" includes a medium storing the program for a predetermined time such as an internal volatile memory (RAM) of a computer system serving as a server or a client in a case in which the program is transmitted through a network. Furthermore, the program described above may be a program used for realizing a part of the function described above. In addition, the program may be a program to be combined with a program that has already been recorded in the computer system for realizing the function described above, a so-called a differential file (differential program).

Furthermore, a part or the whole of the function described above may be realized by an integrated circuit of a large scale integration (LSI) or the like. Each function described above may be individually configured as a processor, or a part or the whole of the functions may be integrated and configured as a processor. In addition, a technique used for configuring the integrated circuit is not limited to the LSI, and each function may be realized by a dedicated circuit or a general-purpose processor. Furthermore, in a case in which a technology of configuring an integrated circuit replacing the LSI emerges in accordance with the progress of semiconductor technologies, an integrated circuit using such a technology may be used.

[Reference Signs List]

| | |
|---|---|
| 1 | Advertisement evaluating system |
| 10 | Data processing apparatus |
| 11 | Display unit |
| 12 | Storage unit |
| 13 | Control unit |
| 20 | Image reproducing terminal |

30    fMRI
40    Corpus
121    Measurement result storing unit
122    Estimation model storing unit
123    Matrix Storing unit
124    Correlation coefficient storing unit
131    Model generating unit
132    Estimation matrix generating unit
133    Intention matrix generating unit
134    Correlation calculating unit
135    Display control unit
S1    Test subject


**Claims**

1.    A viewing material evaluating method comprising:

    a brain activity measuring step of measuring brain activity of a test subject who views a viewing material by using a brain activity measuring unit;
    a first matrix generating step of generating a first matrix estimating semantic content of perception of the test subject on the basis of a measurement result acquired in the brain activity measuring step by using a first matrix generating unit;
    a second matrix generating step of generating a second matrix by performing natural language processing for text information representing a planning intention of the viewing material by using a second matrix generating unit; and
    a similarity calculating step of calculating similarity between the first matrix and the second matrix by using a similarity calculating unit.

2.    The viewing material evaluating method according to claim 1,
    wherein, in the second matrix generating step, the second matrix generating unit translates each of words acquired by dividing the text information into a matrix representing a position in a semantic space of a predetermined number of dimensions and generates the second matrix representing the center of the matrix.

3.    The viewing material evaluating method according to claim 1 or 2,
    wherein cut text information representing a planning intention of each cut included in a storyboard of the viewing material is included in the text information,
    wherein, in the first matrix generating step, the first matrix generating unit generates the first matrix for each cut,
    wherein, in the second matrix generating step, the second matrix generating unit generates the second matrix corresponding to the cut text information, and

wherein, in the similarity calculating step, the similarity calculating unit calculates the similarity for each cut.

4.    The viewing material evaluating method according to any one of claims 1 to 3,
    wherein scene text information representing a planning intention of each scene included in the viewing material is included in the text information,
    wherein, in the first matrix generating step, the first matrix generating unit generates the first matrix for each scene,
    wherein, in the second matrix generating step, the second matrix generating unit generates the second matrix corresponding to the scene text information, and
    wherein, in the similarity calculating step, the similarity calculating unit calculates the similarity for each scene.

5.    The viewing material evaluating method according to any one of claims 1 to 4,
    wherein, in the brain activity measuring step, the brain activity measuring unit measures brain activity of the test subject for each predetermined time interval,
    wherein, in the first matrix generating step, the first matrix generating unit generates the first matrix for each predetermined time interval, and
    wherein, in the similarity calculating step, the similarity calculating unit calculates similarity between a mean first matrix representing a mean of the first matrix in a period corresponding to the text information and the second matrix.

6.    The viewing material evaluating method according to any one of claims 1 to 4,
    wherein overall intention text information representing an overall planning intention of the viewing material is included in the text information,
    wherein, in the brain activity measuring step, the brain activity measuring unit measures brain activity of the test subject for each predetermined time interval,
    wherein, in the first matrix generating step, the first matrix generating unit generates the first matrix for each predetermined time interval,
    wherein, in the second matrix generating step, the second matrix generating unit generates the second matrix corresponding to the overall intention text information, and
    wherein, in the similarity calculating step, the similarity calculating unit calculates the similarity between the first matrix generated for each predetermined time interval and the second matrix corresponding to the overall intention text information.

7.    The viewing material evaluating method according to any one of claims 1 to 6, further comprising:

a training measuring step of measuring brain activity of the test subject viewing a training moving image at a predetermined time interval by using the brain activity measuring unit; and
a model generating step of generating an estimation model for estimating the first matrix from measurement results on the basis of a plurality of the measurement results acquired in the training measuring step and a plurality of third matrixes generated by performing natural language processing for description text describing each scene of the training moving image by using a model generating unit,
wherein, in the first matrix generating step, the first matrix generating unit generates the first matrix on the basis of the measurement result acquired in the brain activity measuring step and the estimation model.

8. A viewing material evaluating system comprising:

a brain activity measuring unit measuring brain activity of a test subject who views a viewing material;
a first matrix generating unit generating a first matrix estimating semantic content of perception of the test subject on the basis of a measurement result acquired by the brain activity measuring unit;
a second matrix generating unit generating a second matrix by performing natural language processing for text information representing a planning intention of the viewing material; and
a similarity calculating unit calculating similarity between the first matrix and the second matrix.

9. A program causing a computer to execute:

a first matrix generating step of generating a first matrix estimating semantic content of perception of a test subject on the basis of a measurement result acquired by a brain activity measuring unit measuring brain activity of the test subject who views a viewing material;
a second matrix generating step of generating a second matrix by performing natural language processing for text information representing a planning intention of the viewing material; and
a similarity calculating step of calculating similarity between the first matrix and the second matrix.

# FIG. 1

# FIG. 2

IMAGE  ⟶  NTT DATA IS YY
AND XX ZZ.
IMPRESSION OF WW...  ⟶  NTT DATA: NOUN, PROPER NOUN, ORGANIZATION, ****
YY: PARTICLE, CASE PARTICLE, COMMON, ***, etc
XX: VERB, SELF-RELIANCE, **, GODAN·WA-LINE
SOKUONBIN, PREDICATIVE FORM, TA-CONNECTION,
USE, TSUKAtsu, TSUKAtsu

P1     P2     P3

⟱

ANNOTATION VECTOR

P4

# FIG. 3

MATRIX SPACE OF N DIMENSIONS

# FIG. 4

## FIG. 5

t1                t2                tn     TIME t

CM MOVING IMAGE ·········

BRAIN ACTIVITY MEASUREMENT RESULT $X_{t1}$ ········· BRAIN ACTIVITY MEASUREMENT RESULT $X_{t2}$ ········· BRAIN ACTIVITY MEASUREMENT RESULT $X_{tn}$

ESTIMATION MATRIX $A_{t1}$ ········· ESTIMATION MATRIX $A_{t2}$ ········· ESTIMATION MATRIX $A_{tn}$

CORRELATION COEFFICIENT $r_{t1}$ ········· CORRELATION COEFFICIENT $r_{t2}$ ········· CORRELATION COEFFICIENT $r_{tn}$

OBJECT CONCEPT VECTOR B (MATRIX B)

TEXT INFORMATION REPRESENTING OVERALL INTENTION OF CM PLANNING PAPER

20

# FIG. 6

```
                    ┌─────────────────┐
                    │      START      │
                    └────────┬────────┘
                             ▼
        ┌──────────────────────────────────────┐
        │     GENERATE ESTIMATION MODEL         │──── S101
        └────────────────────┬─────────────────┘
                             ▼
        ┌──────────────────────────────────────┐
        │    MEASURE BRAIN ACTIVITY VIEWING     │
        │          CM MOVING IMAGE AT           │──── S102
        │     PREDETERMINED TIME INTERVAL       │
        └────────────────────┬─────────────────┘
                             ▼
        ┌──────────────────────────────────────┐
        │    GENERATE ESTIMATION MATRIX A OF    │
        │        EACH PREDETERMINED TIME        │
        │  INTERVAL FROM MEASUREMENT RESULT     │──── S103
        │         AND ESTIMATION MODEL          │
        └────────────────────┬─────────────────┘
                             ▼
        ┌──────────────────────────────────────┐
        │       GENERATE OBJECT CONCEPT         │
        │    VECTOR B (MATRIX B) FROM TEXT       │
        │ INFORMATION REPRESENTING OVERALL      │──── S104
        │  INTENTION OF CM PLANNING PAPER       │
        └────────────────────┬─────────────────┘
                             ▼
        ┌──────────────────────────────────────┐
        │      CALCULATE CORRELATION            │
        │  COEFFICIENT BETWEEN ESTIMATION       │
        │  MATRIX A OF EACH PREDETERMINED       │──── S105
        │     TIME INTERVAL AND OBJECT          │
        │   CONCEPT VECTOR B (MATRIX B)         │
        └────────────────────┬─────────────────┘
                             ▼
        ┌──────────────────────────────────────┐
        │         GENERATE GRAPH OF             │
        │   CORRELATION COEFFICIENT AND         │──── S106
        │  DISPLAY GRAPH ON DISPLAY UNIT        │
        └────────────────────┬─────────────────┘
                             ▼
                    ┌─────────────────┐
                    │       END       │
                    └─────────────────┘
```

# FIG. 7

```
        ┌──────────────┐
        │    START     │
        └──────────────┘
                │
                ▼
  ┌────────────────────────────────┐
  │      MEASURE BRAIN ACTIVITY     │──── S201
  │   VIEWING TRAINING MOVING IMAGE │
  └────────────────────────────────┘
                │
                ▼
  ┌────────────────────────────────┐
  │  GENERATE ANNOTATION VECTOR FROM │──── S202
  │ EACH SCENE OF TRAINING MOVING IMAGE│
  └────────────────────────────────┘
                │
                ▼
  ┌────────────────────────────────┐
  │  GENERATE ESTIMATION MODEL FROM  │
  │   MEASUREMENT RESULT OF BRAIN    │──── S203
  │   ACTIVITY AND ANNOTATION VECTOR │
  └────────────────────────────────┘
                │
                ▼
        ┌──────────────┐
        │     END      │
        └──────────────┘
```

# FIG. 8

# FIG. 9

# FIG. 10

```
                    ┌─────────────┐
                    │    START    │
                    └─────────────┘
                           │
                           ▼
        ┌──────────────────────────────────────┐
        │     GENERATE ESTIMATION MODEL         │──── S301
        └──────────────────────────────────────┘
                           │
                           ▼
        ┌──────────────────────────────────────┐
        │ MEASURE BRAIN ACTIVITY VIEWING CM MOVING │──── S302
        │  IMAGE AT PREDETERMINED TIME INTERVAL │
        └──────────────────────────────────────┘
                           │
                           ▼
        ┌──────────────────────────────────────┐
        │     GENERATE ESTIMATION MATRIX A1     │
        │     OF EACH CUT FROM MEASUREMENT      │──── S303
        │     RESULT AND ESTIMATION MODEL       │
        └──────────────────────────────────────┘
                           │
                           ▼
        ┌──────────────────────────────────────┐
        │      GENERATE OBJECT CONCEPT          │
        │  VECTOR B1 (MATRIX B1) FROM CUT TEXT  │
        │ INFORMATION REPRESENTING INTENTION    │──── S304
        │    OF EACH CUT OF STORYBOARD          │
        └──────────────────────────────────────┘
                           │
                           ▼
        ┌──────────────────────────────────────┐
        │    CALCULATE CORRELATION COEFFICIENT  │
        │ BETWEEN ESTIMATION MATRIX A1 OF EACH CUT │──── S305
        │ AND OBJECT CONCEPT VECTOR B1 (MATRIX B1) │
        └──────────────────────────────────────┘
                           │
                           ▼
        ┌──────────────────────────────────────┐
        │ GENERATE GRAPH OF CORRELATION COEFFICIENT │──── S306
        │   AND DISPLAY GRAPH ON DISPLAY UNIT   │
        └──────────────────────────────────────┘
                           │
                           ▼
                    ┌─────────────┐
                    │     END     │
                    └─────────────┘
```

# FIG. 11

START

GENERATE ESTIMATION MODEL — S401

MEASURE BRAIN ACTIVITY VIEWING CM MOVING
IMAGE AT PREDETERMINED TIME INTERVAL — S402

GENERATE ESTIMATION MATRIX A2
OF EACH SCENE FROM MEASUREMENT
RESULT AND ESTIMATION MODEL — S403

GENERATE OBJECT CONCEPT
VECTOR B2 (MATRIX B2) FROM SCENE TEXT
INFORMATION REPRESENTING INTENTION
OF EACH SCENE OF STORYBOARD — S404

CALCULATE CORRELATION COEFFICIENT
BETWEEN ESTIMATION MATRIX A2 OF EACH SCENE
AND OBJECT CONCEPT VECTOR B2 (MATRIX B2) — S405

GENERATE GRAPH OF CORRELATION COEFFICIENT
AND DISPLAY GRAPH ON DISPLAY UNIT — S406

END

**INTERNATIONAL SEARCH REPORT**

| International application No. |
| --- |
| PCT/JP2016/088375 |

A. CLASSIFICATION OF SUBJECT MATTER
*G06F17/30(2006.01)i*

According to International Patent Classification (IPC) or to both national classification and IPC

B. FIELDS SEARCHED

Minimum documentation searched (classification system followed by classification symbols)
G06F17/30, A61B5/04-A61B5/05, G06Q10/00-50/34

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2014-115913 A (Advanced Telecommunications Research Institute International), 26 June 2014 (26.06.2014), entire text; all drawings & US 2015/0332016 A1 & WO 2014/092045 A1 | 1-9 |
| A | JP 2008-102594 A (Fujitsu Ltd.), 01 May 2008 (01.05.2008), entire text; all drawings & US 2008/0089553 A1 | 1-9 |
| A | JP 2012-73350 A (Canon Inc.), 12 April 2012 (12.04.2012), entire text; all drawings & US 2012/0075530 A1 | 1-9 |

☒ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    23 February 2017 (23.02.17) | Date of mailing of the international search report<br>    07 March 2017 (07.03.17) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/088375

C (Continuation).　DOCUMENTS CONSIDERED TO BE RELEVANT

| Category* | Citation of document, with indication, where appropriate, of the relevant passages | Relevant to claim No. |
|---|---|---|
| A | Yasuhito SAWAHATA, "Neural Decoding During Video Viewing : Towards understanding semantic features of video contents", The Journal of the Institute of Image Information and Television Engineers, 01 July 2015 (01.07.2015), vol.69, no.6, pages 516 to 520 | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016007307 A

- JP 2015077694 A